# EUROPÄISCHE PATENTSCHRIFT

(11) **EP 0 534 992 B1**
(45) Veröffentlichungstag und Bekanntmachung des Hinweises auf die Patenterteilung: **13.09.1995**
(21) Anmeldenummer: 91909262.7
(22) Anmeldetag: 10.05.1991
(51) Int. Cl.: C12P 21/02, C07K 1/02

(54) **VERFAHREN ZUR HERSTELLUNG VON PEPTIDEN**
PROCESS FOR PRODUCING PEPTIDES
PROCEDE DE PRODUCTION DE PEPTIDES

(30) Priorität: 13.06.1990 DD 341603
(43) Veröffentlichungstag der Anmeldung: 07.04.1993
(73) Patentinhaber: HOECHST AKTIENGESELLSCHAFT, 65926 Frankfurt am Main (DE)
(72) Erfinder: JAKUBKE, Hans-Dieter, Talstrasse 33, W-7010 Leipzig (DE); SCHUSTER, Matthias, W-1080 Berlin (DE); AAVIKSAAR, Aavo, Tallinn, 200026 (SU)
(86) Internationale Anmeldenummer: EP9100877
(87) Internationale Veröffentlichungsnummer: WO9119811

(56) Entgegenhaltungen:
- CHEMICAL ABSTRACTS, Band 112, Nr. 25, 18. Juni 1990, Seite 314, Zusammenfassung Nr. 232227p, (Columbus, Ohio, US); M.Y. GOLOLOBOV et al.: "Protease-catalyzed peptide synthesis: effect of temperature on the kinetics of acyl transfer catalyzed by papain", & BIOKHIMIYA (MOSCOW) 1990, 55(2), 338-45, siehe Zusammenfassung
- TETRAHEDRON, Band 46, Nr. 24, 1990, Seiten 8093-8102, Pergamon Press plc (GB); M. SCHUSTER et al.: "Enzyme-catalyzed peptide synthesis in ice", siehe den ganzen Artikel

## Beschreibung

Die Erfindung betrifft ein Verfahren zur Herstellung von Peptiden, die nach Abspaltung der Schutzgruppen als Wirkstoffe bzw. Zwischenproduke von Wirkstoffen dienen können.

### Charakteristik des bekannten Standes der Technik

Zur Herstellung von Peptiden können verschiedene organischchemische Verfahren (vgl. Übersicht: Wünsch, E. (1974) Synthese von Peptiden, in Houben-Weyl, Bd. 15,1/2, Methoden der organischen Chemie, Müller, E. (Hrsg.) Georg Thieme Verlag, Stuttgart) eingesetzt werden. Im Verlauf chemischer Peptidsynthesen werden häufig unerwünschte Nebenreaktionen beobachtet, die die Ausbeute verringern und schwierige und langwierige Reinigungsprozesse erforderlich machen. Ein besonders schwerwiegender Nachteil der herkömmlichen Verfahren ist das ungelöste Problem der Racemisierung, die insbesondere bei Segmentkondensation mittels chemischer Verknüpfungsmethoden in Erscheinung tritt. Da sich Stereoisomere kaum vollständig trennen lassen und die optische Reinheit der Syntheseprodukte für die biologische Aktivität eine notwendige Voraussetzung ist, besitzt die industrielle Synthese von Peptiden mittels organischchemischer Verfahren erhebliche Nachteile. Weiterhin müssen bei allen chemischen peptidsynthetischen Operationen die Drittfunktionen von Aminosäurebausteinen wegen der Gefahr der Nebenreaktionen reversibel maskiert werden. Zur Umgehung der geschilderten Schwierigkeiten bietet sich der Einsatz von Biokatalysatoren für die Katalyse des Peptidknüpfungsschrittes an (vgl. Übersichten: Jakubke, H.-D., u. Kuh, P. (1982) Pharmazie 37 89; Fruton, J.S. (1982) in A. Meister: Adv. Enzymmol. Relat. Areas Mol. Biol. 53, 239; Jakubke, H.-D., Kuh, P. u. Könnecke, A. (1985) Angew. Chem. 97, 79). Durch die Stereospezifität der als Biokatalysator eingesetzten Proteasen bleibt die chirale Integrität erhalten und der hohe Grad der Reaktionskontrolle ermöglicht einen weitgehenden Verzicht auf den Schutz von Drittfunktionen. Im Rahmen der enzymkatalysierten Peptidsynthese kommt der kinetisch kontrollierten Reaktionsführung eine Schlüsselrolle zu. Die bei dieser Methode mit der Peptidproduktbildung einhergehende Hydrolyse des Acylenzymintermediats stellt noch für viele Synthesereaktionen ein Problem dar, indem die Ausbeute an Peptidprodukt begrenzt bleibt.

### Ziel der Erfindung

Ziel der Erfindung ist die Herstellung chiral einheitlicher Peptide bei stark verringerter Hydrolyse des Acylenzymintermediats gegenüber bisher genutzten Methoden.

### Darlegung des Wesens der Erfindung

Der Erfindung liegt die Aufgabe zugrunde, N-Acylaminosäurealkylester in Gegenwart von Proteasen mit Aminosäuren, Aminosäurederivaten bzw. Peptiden mit ungeschüzter N-terminaler alpha-Aminogruppe als Aminokomponente umzusetzen.

Erfindungsgemäß werden Peptide hergestellt aus einer alpha-Aminogruppengeschützten Aminosäure oder einem alpha-Aminogruppen-geschützten Peptid, dessen in Reaktion tretende Carboxylgruppe als Ester vorliegt und einer Aminosäure, einem Aminosäurederivat oder einem Peptid, bei dem die in Reaktion tretende Aminogruppe unblockiert ist, in Gegenwart einer Protease in gefrorener wäßriger Lösung, die gegebenenfalls Anteile organischer Lösungsmittel und/oder Puffersubstanzen enthält. Es werden in hohen Ausbeuten Peptide gebildet, die mit geeigneten chromatorgaphischen oder extraktiven Techniken präparativ separiert werden. Im Gegensatz zu bekannten Synthesen von Peptiden unter Nutzung von Proteasen wird durch das erfindungsgemäße Einfrieren der Reaktionsmischung eine weitaus höhere Peptidausbeute erreicht.

Erstmalig wird für die Stoffklasse der Peptide ihre enzymkatalysierte Synthese in gefrorenen wäßrigen Systemen beschrieben. Der Effekt der Erfindung ist insofern überraschend, als mit einer Temperaturabsenkung und der damit verbundenen Verlangsamung der Reaktionsgeschwindigkeit im allgemeinen eine Ausbeuteverminderung einhergeht, die im wesentlichen durch die Bildung von Nebenprodukten bedingt ist. Unerwartet wird eine hohe Ausbeute an vorzugsweise einem, dem gewünschten, Produkt erreicht.

**Tabelle 1**

| Ausbeutevergleich proteasekatalysierter Peptidsynthesereaktionen in wäßriger flüssiger Phase (25°C) sowie gefrorener wäßriger Phase (-25°C) (Carboxylkomponente) = 2mM, (Aminokomponente) = 50 mM | | | | |
|---|---|---|---|---|
| Carboxylkomp. | Aminokomp. | Enzym | Peptidausbeute (%) | |
| | | | 25°C | -25°C |
| Mal-Phe-Ala-OEtCl | H-Ala-Ala-OH | Papain | 42 | 79 |
| Z-Glu-OMe | H-Ala-Ala-OH | Endoprot. Glu-C | 5 | 76 |
| Mal-Tyr-OMe | H-Ala-Ala-OH | α-Chymotrypsin | 10 | 94 |
| Mal-Tyr-OMe | H-D-Leu-NH₂ | α-Chymotrypsin | 10 | 73 |
| Mal-Tyr-OMe | H-Lys-OH | α-Chymotrypsin | 2 | 44 |
| Mal-Tyr-OMe | H-β-Ala-Gly-OH | α-Chymotrypsin | 13 | 79 |

### Ausführungsbeispiel

In den Beispielen werden die Aminosäuren nach den international gültigen Regeln abgekürzt. Zusätzlich werden folgende Abkürzungen verwendet:
- Z: Benzyloxycarbonyl
- Mal: Maleyl
- OMe: Methylester
- OEtCl: Monochlorethylester
Wenn nicht anders vermerkt, besitzen Aminosäuren bzw. Aminosäurereste mit optisch aktivem Zentrum L-Konfiguration.

### Beispiel 1

### Synthese von Mal-Phe-Ala-Ala-Ala-OH

0,2 ml wäßrige Lösung enthaltend 2 mM Mal-Phe-Ala OEtCl, 50 mM H-Ala-Ala-OH, 25 mM NaOH und 0,15 mg/ml Papain werden tiefgefroren. Danach wird das Reaktionsgemisch bis zum Verbrauch des Mal-Phe-Ala-OEtCl bei -25°C aufbewahrt. Die Ausbeute wird nach dem Auftauen durch RP-HPLC analytisch bestimmt und beträgt 79 % d.Th..

### Beispiel 2

### Synthese von Z-Glu-Ala-Ala-OH

0,2 ml wäßrige Lösung enthaltend 2 mM Z-Glu-OMe, 50 mM H-Ala-Ala-OH, 25 mM NaOH und 5 mg/ml Endoproteinase Glu-C werden tiefgefroren. Danach wird das Reaktionsgemisch bis zum Verbrauch des Z-Glu-OMe bei -25°C aufbewahrt. Die Ausbeute wird nach dem Auftauen durch RP-HPLC analytisch bestimmt und beträgt 76 % d.Th..

### Beispiel 3

### Synthese von Mal-Tyr-Ala-Ala-OH

0,2 ml wäßrige Lösung enthaltend 2 mM Mal-Tyr-OMe, 50 mM H-Ala-Ala-OH, 25 mM NaOH und 0,3 M α-Chymotrypsin werden tiefgefroren. Danach wird das Reaktionsgemisch bis zum Verbrauch des Mal-Tyr-OMe bei -25°C aufbewahrt. Die Ausbeute wird nach dem Auftauen durch RP-HPLC analytisch bestimmt und beträgt 94 % d.Th..

### Beispiel 4

### Synthese von Mal-Tyr-D-Leu-NH

Wie in Beispiel 3, jedoch mit 50 mM H-D-Leu-NH₂ als Aminokomponente. 73 % d.Th. Ausbeute.

### Beispiel 5

### Synthese von Mal-Tyr-Lys-OH

Wie in Beispiel 3, jedoch mit 50 mM H-Lys-OH als Aminokomponente. 44 % d.Th. Ausbeute.

### Beispiel 6

### Synthese von Mal-Tyr-β-Ala-Gly-OH

Wie in Beispiel 3, jedoch mit 50 mM H-β-Ala-Gly-OH als Aminokomponente. 79 % d. Th. Ausbeute.

## Patentansprüche

1. Verfahren zur Herstellung von Peptiden aus Aminosäuren oder entsprechenden Peptiden, die mit einer alpha-Aminoschutzgruppe blockiert sind und deren in Reaktion tretende Carbonylfunktion eine Estergruppierung trägt durch Reaktion mit Aminosäuren, Aminosäurederivaten oder Peptiden mit ungeschützter alpha-Aminofunktion; dadurch gekennzeichnet, daß die Reaktion in gefrorener wäßriger Lösung, die gegebenenfalls organische Lösungsmittelanteile und/oder Puffersubstanzen enthält, unter Katalyse eines Enzyms durchgeführt wird und nach beendeter Kupplung und Aufarbeitung Schutzgruppen partiell oder vollständig entfernt werden.

2. Verfahren nach Anspruch 1, dadurch gekennzeichnet, daß als Enzyme Serin- oder Cysteinproteasen wie α-Chymotrypsin, Endoproteinase Glu-C, Papain o. dgl. verwendet werden.

3. Verfahren nach Anspruch 1 und 2, dadurch gekennzeichnet, daß die enzymkatalysierten Umsetzungen in einem Temperaturbereich von -1°C bis -40°C durchgeführt werden.

4. Verfahren nach Anspruch 1 und 2, dadurch gekennzeichnet, daß anstelle von Puffersubstanzen die Pufferkapazität der in Reaktion tretenden ungeschützten Aminogruppe genutzt wird.

5. Verfahren nach Anspruch 1 bis 4, dadurch gekennzeichnet, daß Enzym an einen Träger gebunden ist.

## Claims

1. A process for preparing peptides from amino acids or corresponding peptides, which are blocked with an alpha-amino protective group and whose carbonyl function entering into the reaction carries an ester group, by reaction with amino acids, amino acid derivatives or peptides with an unprotected alpha-amino function, which comprises carrying out the reaction, using catalysis by an enzyme, in frozen aqueous solution which optionally contains organic solvent constituents and/or buffer substances, and partially or completely removing protective groups after completed coupling and working up.

2. The process as claimed in claim 1, wherein serine proteases or cysteine proteases such as α-chymotrypsin, endoproteinase Glu-C, papain or the like are used as enzymes.

3. The process as claimed in claims 1 and 2, wherein the enzyme-catalyzed reactions are carried out in a temperature range from -1°C to -40°C.

4. The process as claimed in claims 1 and 2, wherein, instead of buffer substances, the buffering capacity of the unprotected amino group entering into the reaction is used.

5. The process as claimed in claims 1 to 4, wherein enzyme is bound to a carrier.

## Revendications

1. Procédé pour la préparation de peptides à partir d'aminoacides ou de peptides correspondants, qui sont bloqués par un groupe protecteur de groupe α-amino et dont la fonction carbonyle entrant en réaction porte un groupement ester, par une réaction avec des aminoacides, dérivés d'aminoacides ou peptides à fonction α-amino non protégée, caractérisé en ce que la réaction est effectuée en solution aqueuse congelée qui contient éventuellement des proportions de solvants organiques et/ou des substances tampons, avec catalyse par une enzyme, et, une fois le couplage et le traitement final terminés, on élimine partiellement ou totalement les groupes protecteurs.

2. Procédé selon la revendication 1, caractérisé en ce que, en tant qu'enzyme, on utilise des sérine ou cystéine protéases telles que l'α-chymotrypsine, l'endoprotéinase Glu-C, la papaïne ou similaire.

3. Procédé selon les revendications 1 et 2, caractérisé en ce que les réactions catalysées par des enzymes sont effectuées dans une plage de températures allant de -1 à -40°C.

4. Procédé selon les revendications 1 et 2, caractérisé en ce que, au lieu de substances tampons, on utilise le pouvoir tampon du groupe amino non protégé entrant en réaction.

5. Procédé selon les revendications 1 à 4, caractérisé en ce que l'enzyme est fixée sur un support.
